(19) Europäisches Patentamt
European Patent Office
Office européen des brevets

(11) Veröffentlichungsnummer: **0 010 518**
**A1**

(12) # EUROPÄISCHE PATENTANMELDUNG

(21) Anmeldenummer: 79810119.2

(22) Anmeldetag: 08.10.79

(51) Int. Cl.³: **C 08 F 20/34**
C 07 D 211/46, C 07 D 211/58
C 07 D 471/10, C 08 L 101/00

(30) Priorität: 13.10.78 CH 10647 78

(43) Veröffentlichungstag der Anmeldung:
30.04.80 Patentblatt 80 9

(84) Benannte Vertragsstaaten:
CH DE FR GB IT

(71) Anmelder: CIBA-GEIGY AG
Patentabteilung Postfach
CH-4002 Basel(CH)

(72) Erfinder: Karrer, Friedrich, Dr.
Rebbergstrasse 5
CH-4800 Zofingen(CH)

(54) **Seitenständige N-heterocyclische Ringe tragende Copolymere, deren Verwendung und Verfahren zu deren Herstellung.**

(57) Seitenständige N-heterocyclische Ringe tragende copolymere Verbindungen mit der wiederkehrenden Struktureinheit der Formel (I)

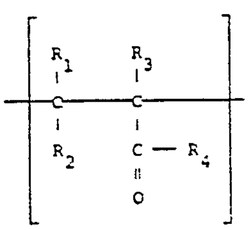

(I)

worin $R_1$ und $R_2$ Wasserstoff sind, und $R_3$ Wasserstoff oder $C_1$-$C_4$ Alkyl bedeutet, und $R_4$ ein N-heterocyclischer Substituent ist, deren Herstellung, deren Verwendung als Lichtschutzmittel und UV-Absorber in organischem Material, sowie das mit deren Hilfe stabilisierte organische Material.

EP 0 010 518 A1

3-12072/S/1+2

BEZEICHNUNG GEÄNDERT
siehe Titelseite

Seitenständige N-heterocyclische Ringe tragende copolymere Verbindungen, deren Verwendung als Lichtschutzmittel und UV-Absorber, und das mit deren Hilfe stabilisierte organische Material.

Die vorliegende Erfindung betrifft neue polymere Verbindungen, deren Herstellung, deren Verwendung als Lichtschutzmittel und UV-Absorber in organischem Material, sowie das mit deren Hilfe stabilisierte organische Material.

Aus dem US Patent Nr. 3,705,166 ist es bekannt, monomere Acrylsäurederivate, welche mindestens eine Piperidinylgruppe mit einem sterisch gehinderten Stickstoffatom enthalten, als Lichtschutzmittel in organischen Polymeren zu verwenden. Diese Acrylsäurederivate sind allerdings zu leicht flüchtig. Ferner wird auf die Möglichkeit hingewiesen, das monomere Additiv in gewisse Substrate einzubauen. Dies hat allerdings den Nachteil, dass die Polymerstruktur durch das eingebaute Additiv gestört ist, was zu schwer kontrollierbarer Veränderung der Eigenschaften des zu schützenden Substrats führen kann. In der JP-OS Nr. 49-75469 sind wasserlösliche Polymere Acryl- und Methacrylsäurederivate als Flockulierungsmittel vorgeschlagen worden.

Ferner sind in US Patent Nr. 3,365,421 homopolymere 2-Hydroxy-4-acryloyläthoxybenzophenone, in US Patent Nr. 3,399,173 homopolymere 2-(2'-Hydroxyphenyl)-benztriazole und aus US Patent Nr. 3,943,094 polymere Alkenoyloxybenzyliden-malonsäureester als UV-Absorber beschrieben worden.

Es wurden nun copolymere Additive gefunden, welche neben ausgezeichneten lichtstabilisierenden Eigenschaften, guter Löslichkeit bzw. Verträglichkeit im zu schützenden Polymer, hoher Extraktions-

BAD ORIGINAL

und Migrationsbeständigkeit gleichzeitig hohe UV-Absorber Wirkung aufweisen.

Die vorliegende Erfindung betrifft seitenständige N-heterocyclische Ringe tragende copolymere Verbindungen mit der wiederkehrenden Struktureinheit der Formel (I)

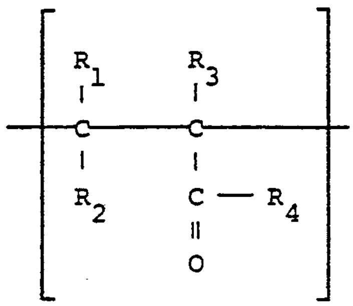

(I) ,

worin $R_1$ und $R_2$ Wasserstoff sind, und $R_3$ Wasserstoff oder $C_1$-$C_4$ Alkyl bedeutet, und $R_4$ ein N-heterocyclischer Substituent der Formeln (II), (III), (IV) oder (V)

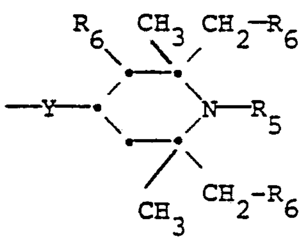

,

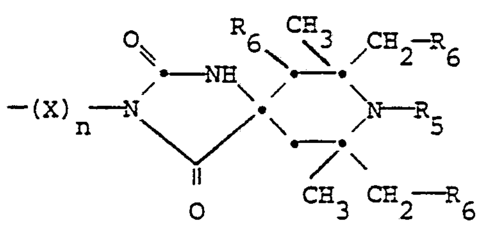

(II) (III)

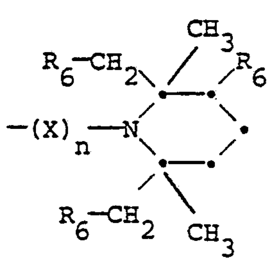

,

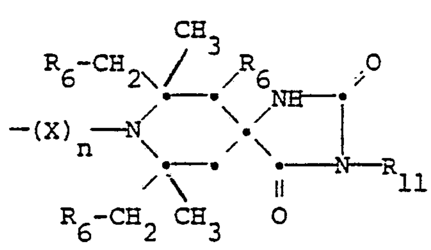

(IV) (V)

bedeutet, worin $R_5$ Wasserstoff, Oxyl, $C_1$-$C_{18}$ Alkyl, $C_3$-$C_8$ Alkenyl, $C_3$-$C_6$ Alkinyl, $C_7$-$C_{12}$ Aralkyl, $C_2$-$C_{21}$ Alkoxyalkyl, Cyanomethylen, eine aliphatische Acylgruppe mit 1 bis 4 Kohlenstoffatomen oder eine Gruppe $-CH_2COOR_7$ ist, wobei $R_7$ $C_1$-$C_{12}$ Alkyl, $C_3$-$C_8$ Alkenyl, Phenyl, $C_7$-$C_8$ Aralkyl oder Cyclohexyl bedeutet, und $R_6$ Wasserstoff oder Methyl bedeutet, und Y -O- oder $-\overset{|}{N}-R_9$ ist, wobei $R_9$ Wasserstoff, $C_1$-$C_{12}$ Alkyl, $C_5$-$C_8$ Cycloalkyl, $C_7$-$C_{12}$ Aralkyl ist, und X eine Gruppe der Formel $-O-CH(R_{10})-CH_2-$ (VI) ist, worin $R_{10}$ Wasserstoff, Methyl, Aethyl, Phenoxymethyl oder Phenyl bedeutet, und n 0 oder 1 ist, und $R_{11}$ $C_1$-$C_{18}$ Alkyl, Cyclohexyl, Phenyl, Benzyl oder eine Gruppe der Formel $-CH_2-COOR_{12}$ (VII) bedeutet, wobei $R_{12}$ $C_1$-$C_8$ Alkyl ist, und ferner $R_4$ eine der Gruppen der Formeln (VIII), (IX), (X) oder (XI)

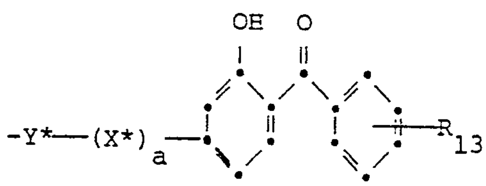

(VIII)

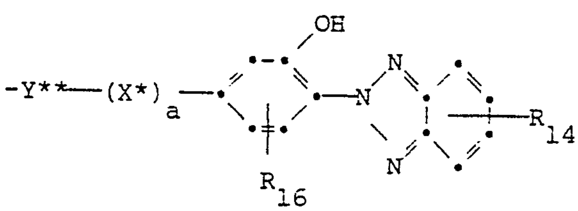

(IX)

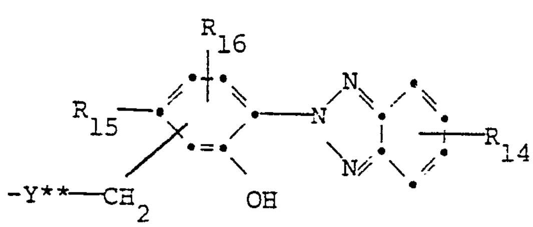

(X)

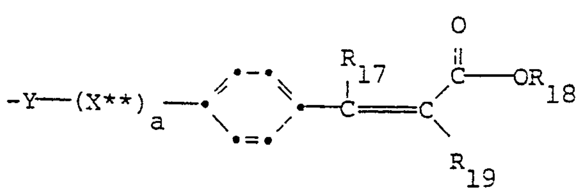

(XI)

bedeutet, worin Y und Y** die oben für Y definierte Bedeutung haben, und Y* -O- oder $-\overset{|}{N}-R_9$* ist, wobei $R_9$* Wasserstoff oder $C_1$-$C_8$ Alkyl ist, und X* eine Gruppe der Formel $-CH(R_{10})-(CH_2)_b-O-$ (XII) bedeutet, b 1 oder 2 ist, und $R_{10}$ die oben angegebene Bedeutung hat, und a O oder 1 ist, und $R_{13}$ Wasserstoff, Methyl oder Chlor bedeutet, und $R_{14}$ Wasserstoff, Methyl, Methoxy oder Chlor bedeutet, und $R_{15}$ Wasserstoff oder Hydroxy bedeutet, und $R_{16}$ Wasserstoff, $C_1$-$C_{12}$ Alkyl, $C_5$-$C_8$ Cycloalkyl, Phenyl, $C_7$-$C_{12}$ Aralkyl oder Chlor ist, und X** eine Gruppe der Formel (XII) ist, worin $R_{10}$ die oben angegebene Bedeutung hat, und b 1 ist, und $R_{17}$ Wasserstoff oder $C_1$-$C_4$ Alkyl bedeutet, und $R_{18}$ $C_1$-$C_{12}$ Alkyl, $C_5$-$C_8$ Cycloalkyl oder eine Gruppe der Formel (XIII)

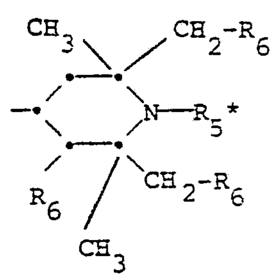

(XIII) ,

worin $R_5$* Wasserstoff, $C_1$-$C_8$ Alkyl, $C_3$-$C_8$ Alkenyl, $C_7$-$C_{12}$ Aralkyl oder eine aliphatische Acylgruppe mit 1 bis 4 Kohlenstoffatomen ist, und $R_6$ die oben angegebene Bedeutung hat, und $R_{19}$ -CN oder eine Gruppe der Formel $-COOR_{18}$ (XIV) ist, worin $R_{18}$ die oben angegebene Bedeutung hat, mit der Bedingung, dass im copolymeren Molekül mit der wiederkehrenden Struktureinheit I der Substituent $R_4$ mindestens einmal (A) eine der Gruppen der Formel (II), (III), (IV) oder (V) und mindestens einmal (B) eine der Gruppen der Formeln (VIII), (IX), (X) oder (XI) bedeutet, wobei (A):(B) im Verhältnis von 50:1 bis 1:50 stehen. $R_3$ kann als $C_1$-$C_4$ Alkyl z.B. Methyl, Aethyl, n-Propyl oder n-Butyl sein; bevorzugt bedeutet $R_3$ Wasserstoff oder Methyl. $R_4$ kann eine der Gruppen der Formeln (II), (III), (IV), (V), (VIII), (IX), (X) oder (XI) sein. Bevorzugt sind Gruppen der Formeln (II), (IV), (VIII), (IX) und (X).

$R_5$ ist als $C_1$-$C_{18}$ Alkyl z.B. Methyl, Aethyl, n-Propyl, n-Butyl, n-Pentyl, n-Octyl, n-Decyl, n-Dodecyl oder Octadecyl. Bevorzugt

BAD ORIGINAL

sind Alkylgruppen mit 1 bis 12, ferner solche mit 1 bis 8, insbesondere solche mit 1 bis 4 Kohlenstoffatomen und vor allem Methyl.

$R_5$ ist als $C_3$-$C_8$ Alkenyl beispielsweise Allyl, 3-Methyl-2-butenyl, 2-Butenyl, 2-Hexenyl oder 2-Octenyl, insbesondere Allyl.

$R_5$ ist als $C_3$-$C_6$ Alkinyl z.B. Propargyl.

$R_5$ ist als $C_7$-$C_{12}$ Aralkyl z.B. Benzyl, β-Phenyl-äthyl oder 4-tert. Butyl-benzyl, bevorzugt Benzyl.

Bedeutet $R_5$ $C_2$-$C_{21}$ Alkoxyalkyl, so kann der Alkylteil 1 bis 3 Kohlenstoffatome enthalten und der Alkoxy-Teil aus 1 bis 18 Kohlenstoffatomen bestehen, wie z.B. in Methoxymethyl, Aethoxymethyl, 2-Methoxyäthyl, 2-Aethoxyäthyl, 2-n-Butoxyäthyl, 3-n-Butoxypropyl, 2-Octoxyäthyl oder 2-Octadecyloxyäthyl; insbesondere zu erwähnen sind Verbindungen, in denen $R_5$ eine Alkoxyalkylgruppe mit 2 bis 6 Kohlenstoffatomen bedeutet.

$R_5$ ist als aliphatische Acylgruppe mit 1 bis 4 Kohlenstoffatomen, beispielsweise Formyl, Acetyl, Acryloyl oder Crotonoyl, insbesondere Acetyl.

Ist $R_5$ die Gruppe $-CH_2COOR_7$ so bedeutet $R_7$ als $C_1$-$C_{12}$ Alkyl, z.B. Methyl, Aethyl, Isopropyl, n-Butyl, Isobutyl, t-Butyl, Isopentyl, n-Octyl oder n-Dodecyl. Bevorzugt ist $R_7$ $C_1$-$C_4$ Alkyl. $R_7$ ist als $C_3$-$C_8$ Alkenyl z.B. Allyl, 2-Butenyl oder 2-Hexenyl. $R_7$ ist als $C_7$-$C_8$ Aralkyl z.B. Benzyl oder α-Phenyläthyl. $R_6$ kann Methyl oder Aethyl und bevorzugt Wasserstoff bedeuten, Y bedeutet $-\overset{|}{N}-R_9$ und bevorzugt -O-.

$R_9$ und $R_{18}$ sind als $C_1$-$C_{12}$ Alkyl z.B. Methyl, Aethyl, n-Propyl, n-Butyl, n-Pentyl, n-Octyl, n-Decyl oder n-Dodecyl, bevorzugt jedoch $C_1$-$C_4$ Alkyl.

$R_9$ und $R_{16}$ ist als $C_7$-$C_{12}$ Aralkyl insbesondere α,α-Dimethylbenzyl, Benzyl oder vor allem α-Methylbenzyl. $R_9$,$R_{16}$ und $R_{18}$ sind als $C_5$-$C_8$ Cycloalkyl z.B. Cyclopentyl, Cycloheptyl, Cyclooctyl und bevorzugt Cyclohexyl. $R_{10}$ ist Phenyl oder Phenoxymethyl, bevorzugt Methyl, Aethyl und insbesondere Wasserstoff. $R_{11}$ ist als $C_1$-$C_{18}$ Alkyl z.B. Methyl, Aethyl, Propyl, Butyl, Hexyl, Octyl, Dodecyl oder Octadecyl. Bevorzugte Alkylgruppen sind solche mit 1 bis 12 Kohlenstoffatomen. $R_{11}$ kann auch Benzyl, Cyclohexyl oder Phenyl bedeuten.

$R_9$* und $R_{12}$ sind als $C_1$-$C_8$ Alkyl z.B. Methyl, Aethyl, n-Propyl, n-Butyl, n-Hexyl oder n-Octyl, $R_9$* besitzt bevorzugt 1 bis 4 Kohlenstoffatome. Y* ist bevorzugt -O-.

$R_{13}$, $R_{14}$ und $R_{15}$ sind in bevorzugten Verbindungen Wasserstoff. In bevorzugten Verbindungen ist $R_{13}$ in 4'-Stellung, währenddem $R_{14}$ vorzugsweise in 5 Stellung steht. $R_{16}$ kann Alkyl mit 1 bis 12, bevorzugt mit 1 bis 8 Kohlenstoffatomen bedeuten, wie Methyl, Aethyl, iso-Propyl, iso-Butyl, t.-Butyl, iso-Pentyl, t-Amyl, Hexyl, 1,1,3,3-Tetramethylbutyl, oder 1,1,3,3,5,5-Hexamethylhexyl. $R_{16}$ ist in bevorzugten Verbindungen in 5'-Stellung gebunden, während die Gruppe $-CH_2$-Y**- vorzugsweise in 3'-Stellung am 2-(2'-Hydroxyphenyl)-benztriazolsystem steht, oder auch in 5'-Stellung falls diese nicht durch einen Substituenten $R_{16}$ besetzt ist.

$R_{17}$ kann als $C_1$-$C_4$ Alkyl, Aethyl, n-Propyl oder n-Butyl sein; bevorzugt aber Methyl.

$R_{17}$ ist bevorzugt Alkyl, falls $R_{19}$ -CN bedeutet; und ist bevorzugt Wasserstoff, falls $R_{19}$ eine Gruppe der Formel (XIV) ist.

Bedeutet $R_{18}$ eine Gruppe der Formel (XIII) so kann $R_5$* in den gegebenen Grenzen die Bedeutungen, wie sie oben für $R_5$ definiert wurden, annehmen.

Bei den Verbindungen mit der wiederkehrenden Struktureinheit der Formel (I) handelt es sich um Copolymere, welche durch Copolymerisation von mindestens zwei monomeren Komponenten gewonnen werden und zwar dergestalt, dass $R_4$ im makromolekularen Molekül mindestens einmal (A) eine der Gruppen der Formel (II), (III), (IV) oder (V) und mindestens einmal (B) eine der Gruppen der Formeln (VIII), (IX), (X) oder (XI) bedeutet, wobei (A):(B) in einem molekularen Verhältnis von 50:1 bis 1:50 stehen . Das Verhältnis der Stuktureinheiten, worin $R_4$ eine Gruppe der Formel (II), (III), (IV) oder (V) ist (A) zu denjenigen, worin $R_4$ eine Gruppe der Formel (VIII), (IX), (X) oder (XI) (B) ist, beträgt bevorzugt 10:1 bis 1:10 und insbesondere 5:1 bis 1:5 und besonders bevorzugt 3:1 bis 1:3.

Selbstverständlich können auch Copolymere aus mehr als zwei monomeren Komponenten, z.B. Terpolymere verwendet werden.

Die in den Terpolymeren vorkommende dritte Komponente kann obenstehende Bedingung A) oder B) erfüllen oder es kann sich um andere polymerisierbare Monomere handeln. Dadurch kann auf einfache Weise ein Einfluss auf die Eigenschaften, wie die Löslichkeit oder den Erweichungspunkt des Additivs genommen werden. Als copolymerisierbare Terkomponenten eignen sich daher beispielsweise Styrol, Divinylbenzol, 2- oder 4-Vinylpyridin, Verbindungen aus der Acrylsäurereihe, wie Ester oder Amide, die sich von Acrylsäure oder Methacrylsäure ableiten, z.B. Methylacrylat, Butylacrylat, Methylmethacrylat, Acrylnitril, Methacrylnitril, Acrylsäureglycidylester, Methylenbisacrylamid oder Aethylenglykoldimethylacrylat; oder es kann sich um 1-Alkene mit 2-10 C-Atomen, z.B. Aethylen, Propylen, 1-Buten, 1-Penten, 1-Hexen, 1-Octen oder 1-Decen handeln; ferner kann es sich um Isopren, Butadien, Vinylester, wie Essigsäurevinylester, um Vinyläther, N-Vinyl-pyrrolidon-2, N-Vinylcarbazol, Maleinimide oder ungesättigte Phosphonate handeln. Bevorzugte copolymerisierbare Komponenten sind Styrol, Acrylnitril, Acryl- oder Methacrylsäureester, Vinylester, Vinyläther oder Acryl- bzw. Methacrylamide.

Das Molekulargewicht der erfindungsgemässen Copolymeren beträgt vorzugsweise mehr als 500; es kann aber durchaus bis zu 150'000 ausmachen. Bevorzugte Verbindungen besitzen ein Molekulargewicht von 500 bis 60'000 und insbesondere von 1000 bis 20'000.

Hervorzuheben sind copolymere Verbindungen mit der wiederkehrenden Struktureinheit der Formel (I), worin $R_1$ und $R_2$ Wasserstoff sind, und $R_3$ Wasserstoff oder Methyl ist, und $R_4$ eine Gruppe der Formeln (II), (III), (IV) oder (V) ist, worin $R_5$ Wasserstoff, $C_1$-$C_{12}$ Alkyl, Allyl, Propargyl, Benzyl, Acetyl, oder $C_2$-$C_6$ Alkoxyalkyl bedeutet, und $R_6$ Wasserstoff oder Methyl ist, und Y -O- oder $-\overset{|}{N}-R_9$ ist, worin $R_9$ Wasserstoff, $C_1$-$C_{12}$ Alkyl, Cyclohexyl oder Benzyl ist und X eine Gruppe der Formel (VI) bedeutet, worin $R_{10}$ Wasserstoff, Methyl oder Aethyl ist, und n 0 oder 1 ist, und $R_{11}$ $C_1$-$C_{12}$ Alkyl, Allyl oder Benzyl bedeutet, und ferner $R_4$ eine Gruppe der Formeln (VIII), (IX), (X) oder (XI) bedeuten, worin Y und Y** die

oben für Y angegebene Bedeutung haben, und Y* -O- oder $-\overset{|}{N}-R_9{}^*$ ist, worin $R_9{}^*$ Wasserstoff oder $C_1$-$C_4$ Alkyl ist, und X* eine Gruppe der Formel (XII) ist, worin $R_{10}$ die oben definierte Bedeutung hat, und b 1 oder 2 ist, und a O oder 1 ist, $R_{13}$ Wasserstoff, Methyl oder Chlor ist, und $R_{14}$ und $R_{15}$ Wasserstoff sind, und $R_{16}$ Wasserstoff, $C_1$-$C_8$ Alkyl, Cyclohexyl, Phenyl, $C_7$-$C_9$ Aralkyl oder Chlor ist, und X** und $R_{17}$ die oben definierte Bedeutung haben und $R_{18}$ $C_1$-$C_8$ Alkyl, Cyclohexyl oder eine Gruppe der Formel (XIII) ist, worin $R_5{}^*$ Wasserstoff, $C_1$-$C_8$ Alkyl, Allyl, Benzyl oder Acetyl ist, und $R_6$ die angegebene Bedeutung hat, und $R_{19}$ -CN oder eine Gruppe der Formel (XIV) ist, worin $R_{18}$ die oben angegebene Bedeutung hat,
mit der Bedingung, dass im copolymeren Molekül mit der wiederkehrenden Struktureinheit I $R_4$ mindestens einmal (A) eine der Gruppen (II), (III), (IV) oder (V) und mindestens einmal (B) eine der Gruppen der Formeln (VIII), (IX), (X) oder (XI) bedeutet, wobei (A):(B) im Verhältnis von 50:1 bis 1:50 stehen.

Bevorzugt sind copolymere Verbindungen mit der wiederkehrenden Struktureinheit der Formel (I), worin $R_1$ und $R_2$ Wasserstoff sind, und $R_3$ Wasserstoff oder Methyl bedeutet, und $R_4$ eine Gruppe der Formeln (II) oder (IV) ist, worin $R_5$ Wasserstoff, $C_1$-$C_8$ Alkyl, Allyl, Benzyl oder Acetyl ist, und $R_6$ Wasserstoff bedeutet, und Y -O- oder $-\overset{|}{N}-R_9$ ist, worin $R_9$ Wasserstoff oder $C_1$-$C_4$ Alkyl bedeutet, und X eine Gruppe der Formel (VI) ist, worin $R_{10}$ Wasserstoff oder Methyl und n O oder 1 bedeutet, und ferner $R_4$ eine Gruppe der Formeln (VIII), (IX), (X) oder (XI) ist, worin Y die oben angegebene Definition besitzt, und Y** $-\overset{|}{N}-R_9$ ist, worin $R_9$ die oben angegebene Bedeutung hat, und Y* -O- ist, und X* und X** eine Gruppe der Formel (XII) ist, worin $R_{10}$ die oben angegebene Bedeutung hat und b 1 ist, und a O oder 1 ist, und $R_{13}$, $R_{14}$ und $R_{15}$ Wasserstoff sind, und $R_{16}$ H, $C_1$-$C_8$ Alkyl oder $C_7$-$C_9$ Aralkyl bedeutet, und $R_{17}$, falls $R_{19}$ -CN ist, $C_1$-$C_4$ Alkyl oder, falls $R_{19}$ eine Gruppe der Formel (XIV) ist, Wasserstoff bedeutet, und $R_{18}$ $C_1$-$C_8$ Alkyl oder eine Gruppe der Formel (XIII) bedeutet, worin $R_5{}^*$

Wasserstoff, $C_1$-$C_4$ Alkyl, Benzyl oder Acetyl ist, und $R_4$ die oben erwähnte Definition besitzt, und $R_{19}$ -CN oder eine Gruppe der Formel (XIV) ist, worin $R_{18}$ die oben erwähnte Bedeutung hat, mit der für $R_4$ oben erwähnten Bedingung.

Bevorzugt sind auch copolymere Verbindungen mit der wiederkehrenden Struktureinheit der Formel (I), worin $R_1$ und $R_2$ Wasserstoff sind, und $R_3$ Wasserstoff oder Methyl ist, und $R_4$ eine Gruppe der Formeln (II) oder (IV) ist, worin $R_5$ Wasserstoff, $C_1$-$C_4$ Alkyl, Benzyl oder Acetyl ist, und $R_6$ Wasserstoff bedeutet, und Y -O- oder -N-$R_9$ ist, worin $R_9$ Wasserstoff oder $C_1$-$C_4$ Alkyl ist, und X eine Gruppe der Formel (VI) ist, worin $R_{10}$ Wasserstoff oder Methyl und n 0 oder 1 ist, und $R_4$ eine Gruppe der Formeln (VIII), (X) oder (XI) ist,worin Y und Y* -O- sind, und Y** -N-$R_9$ ist, wobei $R_9$ die oben angegebene Bedeutung hat, und X* und X** eine Gruppe der Formel (XII) sind, worin $R_{10}$ die oben angegebene Bedeutung hat, und b 1 ist, und a 0 oder 1 ist, und $R_{13}$, $R_{14}$ und $R_{15}$ Wasserstoff sind, und $R_{16}$ H, $C_1$-$C_8$ Alkyl oder $C_7$-$C_9$ Aralkyl ist, und $R_{17}$, falls $R_{19}$ -CN ist, Methyl oder, falls $R_{19}$ eine Gruppe der Formel (XIV) ist, Wasserstoff bedeutet, und $R_{18}$ $C_1$-$C_8$ Alkyl oder eine Gruppe der Formel (XIII) ist, worin $R_5$* Wasserstoff oder Methyl bedeutet, und $R_6$ Wasserstoff ist, und $R_{19}$ -CN oder eine Gruppe der Formel (XIV) bedeutet, worin $R_{18}$ die oben erwähnte Bedeutung hat, mit der für $R_4$ oben erwähnten Bedingung.

Besonders bevorzugt sind copolymere Verbindungen mit der wiederkehrenden Struktureinheit der Formel (I), worin $R_1$ und $R_2$ Wasserstoff sind, und $R_3$ Wasserstoff oder Methyl ist, und $R_4$ eine Gruppe der Formeln (II) oder (IV) ist, worin $R_5$ Wasserstoff, Methyl oder Acetyl bedeutet, und $R_6$ Wasserstoff ist, und Y -O- oder -N-$R_9$ ist, worin $R_9$ Wasserstoff oder $C_1$-$C_4$ Alkyl bedeutet,und X eine Gruppe der Formel(VI) ist, worin $R_{10}$ Wasserstoff und n 0 oder 1 bedeutet, und ferner $R_4$ eine Gruppe der Formeln (VIII) oder (X) ist, worin Y* -O- ist, und Y** -N-$R_9$ ist, wobei $R_9$ die oben angegebene Bedeutung hat, und X* und X** eine Gruppe der Formel (XII) sind, worin $R_{10}$ die oben angegebene Bedeutung hat, und b 1 ist, und $R_{13}$, $R_{14}$ und $R_{15}$ Wasserstoff sind, und

BAD ORIGINAL

$R_{16}$ H, $C_1$-$C_8$ Alkyl oder $C_7$-$C_9$ Aralkyl bedeutet, mit der oben für $R_4$ erwähnten Bedingung.

Die Herstellung der Copolymeren mit der wiederkehrenden Struktureinheit der Formel (I) erfolgt nach bekannten Polymerisationsreaktionen, welche beispielsweise in Houben-Weyl, 14 (1) 1010-1078 (1962) beschrieben sind. Es wird dabei z.B. ein Monomer der Formel (XVa)

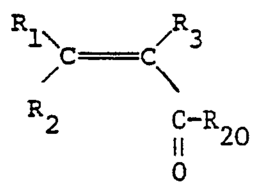

(XVa)

mit einem Monomer der Formel (XVb)

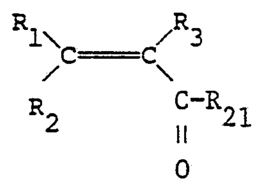

(XVb) ,

copolymerisiert,

wobei $R_{20}$ eine Gruppe der oben definierten Formeln (II), (III), (IV) oder (V) ist, und $R_{21}$,eine Gruppe der oben definierten Formeln (VIII), (IX), (X) oder (XI) ist, wobei das Verhältnis von Monomer (XVa) zu Monomer (XVb) 50:1 bis 1:50 betragen soll. In den Formeln (XVa) und (XVb) haben die Substituenten $R_1$, $R_2$ und $R_3$ die oben angegebene Bedeutung.

Die Herstellung der zur Polymerisation eingesetzten Monomere der Formel (XVa) erfolgt auf an sich bekannte Weise, z.B. analog der im US Patent No. 3,705,166 beschriebenen Methoden. Es wird dabei beispielsweise ein reaktionsfähiges Derivat einer ungesättigten Carbonsäure der Formel (XVI)

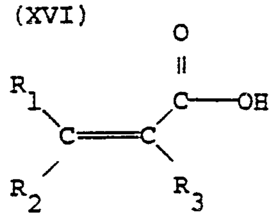

(XVI)

0010518

mit einer der Verbindungen der Formeln (II*), (III*), (IV*) oder (V*)

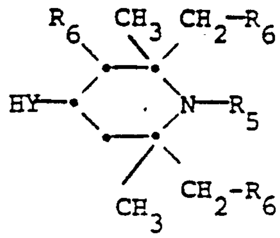

,

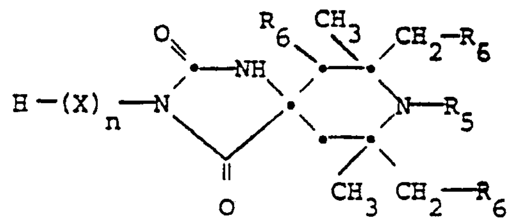

(II*)

(III*)

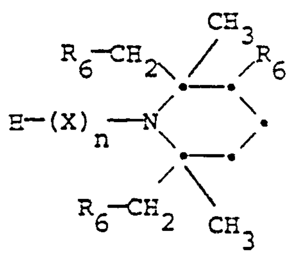

,

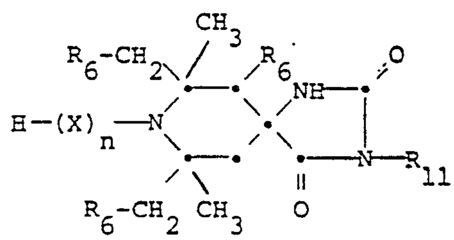

(IV*)

(V*)

worin alle Substituenten oben definierte Bedeutung haben, vorzugsweise in einem inerten organischen Lösungsmittel umgesetzt.

Reaktionsfähige Derivate einer ungesättigten Carbonsäure sind beispielsweise ein Säurehalogenid der Formel (XVIa)

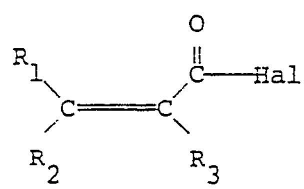

(XVIa) ,

worin Hal, Brom oder insbesondere Chlor bedeutet, oder ein Säureanhydrid der Formel (XVIb)

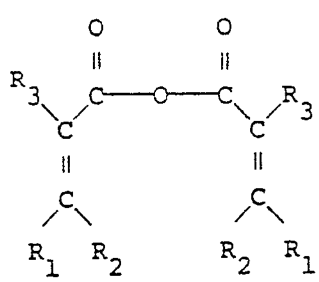

. (XVIb)

Pro Mol einer der Verbindungen der Formel (II*), (III*), (IV*) oder ( V*) wird vorzugsweise ungefähr ein Mol einer Verbindung der Formel (XVIa) oder (XVIb) eingesetzt. $R_1$, $R_2$ und $R_3$ in den Formeln (XVIa) und (XVIb) entsprechen der oben angegebenen Definition.

Beim Verwenden eines Säurehalogenids der Formel (XVIa) arbeitet man in Gegenwart einer Base z.B. in Gegenwart eines tert.Amins, wie Triäthylamin, Di-isopropyl-äthylamin, N,N-Diäthylanilin oder Pyridin; oder in Gegenwart eines wasserfreien Alkalimetall- oder Erdalkalimetallcarbonats, oder Alkalimetallbicarbonats, wie $MgCO_3$, $NaHCO_3$, $Na_2CO_3$ oder $K_2CO_3$. Wird anstelle des Säurechlorids ein Säureanhydrid der Formel (XVIb) verwendet, so kann gegebenenfalls auf die Base verzichtet werden.

Die bei den oben beschriebenen Verfahrensvarianten verwendeten organischen Lösungsmittel müssen gegenüber den Reaktionspartnern inert sein. Es eignen sich dabei beispielsweise aliphatische Kohlenwasserstoffe wie Hexan oder Ligroin; aromatische Kohlenwasserstoffe, wie Benzol, Toluol oder Xylol; chlorierte Kohlenwasserstoffe, wie Methylenchlorid, Chloroform oder 1,2-Dichloräthan; Amide, wie Hexamethylenphosphorsäuretriamid; oder Aether wie Dioxan, 1,2-Dimethoxyäthan, Diäthyläther oder Tetrahydrofuran.

Die Temperatur dieser Reaktion beträgt vorzugsweise -20 bis +120° C, insbesondere aber -10° C bis +80° C.

Eine weitere Verfahrensvariante besteht darin, dass man als reaktionsfähiges Derivat einer Carbonsäure der Formel (XVI) einen Ester der Formel (XVIc)

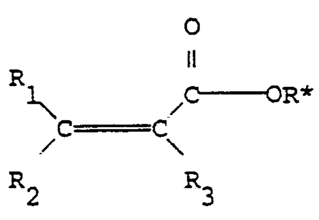

(XVIc) ,

worin $R_1$, $R_2$ und $R_3$ die oben angegebene Bedeutung haben und R* $C_1$-$C_4$ Alkyl ist, verwendet. Die Reaktionspartner der Formeln (II*), (III*), (IV*) oder (V*), worin n 1 ist und die übrigen Symbole die oben angegebene Bedeutung haben, können in ungefähr stöchiometrischen Mengen oder mit einem Ueberschuss an (XVIc) zur Reaktion gebracht werden.

Es handelt sich dabei um eine übliche Umesterungsmethode, welche bei erhöhter Temperatur, mit oder ohne Lösungsmittel und in Gegenwart eines Umesterungskatalysators, z.B. einer Säure oder vorzugsweise einer Base wie Titantetrabutyrat, Titantetrapropionat, Aluminiumisopropoxid, Lithiumamid, Lithiumhydrid oder Natriumhydrid stattfindet. Die Temperatur beträgt vorzugsweise 20 bis 170°C, insbesondere 50 bis 150°C. Wird in einem Lösungsmittel gearbeitet, so kann eines der oben angeführten verwendet werden.

Es kann auch ein Ionenaustauschharz als Katalysator eingesetzt werden. Da es sich bei den Verbindungen der Formel (XVIc) um flüssige Verbindungen handelt, kann gegebenenfalls auf ein Lösungsmittel verzichtet werden. Die Reaktionskomponente (XVIc) ist gegebenenfalls vor der Umesterungsreaktion mit einem der bekannten Stabilisatoren, wie z.B. Hydrochinon, Hydrochinonmonomethyläther, 2,6-Di-tert.-butyl-p-kresol, einem anderen 2,6-Di-tert.-butyl-phenyl-Derivat oder Phenothiazin, zu stabilisieren.

Die als Ausgangsstoffe verwendeten Verbindungen der Formeln (XVI), (XVIa), (XVIb) und (XVIc) sind dem Fachmann bekannte Verbindungen und können, falls nicht im Handel erhältlich auf einfache Weise hergestellt werden.

Die als Reaktionspartner verwendeten Piperidinylderivate sind ebenfalls bekannte Verbindungen. Die Herstellung der Verbindungen der Formel (II*), ist beispielsweise in der DE-OS 2,352,658 (4-Hydroxypiperidine) oder in dem US Patent Nr. 3,684,765 (4-Aminopiperidine) beschrieben worden.

Die Herstellung der Verbindungen der Formeln (III*) und (V*) kann analog der in DE-OS 2,227,689 beschriebenen Methoden erfolgen.

Die Herstellung von Verbindungen der Formel (IV*) ist beispielsweise aus der DE-OS 2,418,540 bekannt.

Die Verbindungen derFormeln (II*), (III*), (IV*) und (V*), welche im Piperidylring in 2- und 6-Stellung verschiedenartige Substituenten besitzen, können durch Umsetzung eines Ketones der Formel $CH_3$-CO-$CH_2$-$R_6$ mit Ammoniak hergestellt werden. Das gebildete Pyrimidin wird, wie in Helv. Chim. Acta 30, 114 (1947) beschrieben, zu einem Aminoketon der Formel (XVII) hydrolysiert.

BAD ORIGINAL

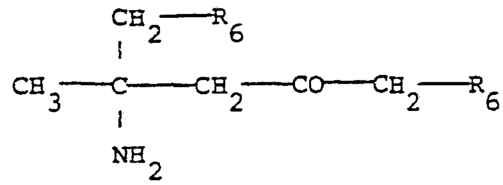 (XVII)

Die Verbindungen der Formel (XVII) werden in einem zweiten Verfahrensschritt mit Ammoniak und einem Keton $CH_3$-CO-$CH_2$-$R_6$ umgesetzt, wie es z.B. in Monatsh. Chemie 88, 464 (1957) beschrieben ist. $R_6$ hat in den angegebenen Formeln die oben genannte Bedeutung. Die Verbindungen der Formeln (II*) und (III*), worin $R_5$ Wasserstoff bedeutet, können aus dem so erhaltenen Pyrimidin durch Hydrolyse gewonnen werden.

Die Verbindungen, welche in 1- und/oder 4-Stellung von Wasserstoff verschiedene Substituenten tragen, werden analog der in den oben angegebenen Literaturstellen beschriebenen Methoden hergestellt.

Die Herstellung der zur Copolymerisation eingesetzten Monomere der Formel (XVb) erfolgt ebenfalls auf bekannte Weise.

So ist beispielsweise im US Patent Nr. 3,365,421 die Herstellung der Monomeren der Formel (XVb), worin $R_{21}$ eine Gruppe der Formel (VIII) ist, beschrieben. Ueblicherweise gelangt man zu diesen Verbindungen durch Veresterung von Acryl- bzw. Methacrylsäure mit den entsprechenden 2-Hydroxy-4-(2'-hydroxyäthoxy)- bzw. 2,4-Dihydroxy-benzophenonen.

Die Benztriazolderivate der Formel (XVb), worin $R_{21}$ eine Gruppe der Formel (IX) oder (X) ist, werden analog den in US Patent Nr. 3,399,173 beschriebenen Methoden hergestellt.

Die Herstellung der Monomere der Formel (XVb), worin $R_{21}$ eine Gruppe der Formel (XI) bedeutet, ist ausführlich z.B. in Org. Reactions, Vol. XV, 332 (1967) oder im US Patent Nr. 3,943,094 beschrieben.

Monomere der Formel (XVa), welche sich für die Herstellung der erfindungsgemässen copolymeren Additive eignen, sind z.B.

1,2,2,6,6-Pentamethyl-4-acryloyloxy-piperidin
1-Methacryloyloxyäthyl-2,2,6,6-tetramethyl-piperidin
1-Acryloyloxyäthyl-2,2,6,6-tetramethyl-piperidin
1,2,2,6,6-Pentamethyl-4-maleinimido-piperidin

1-Acetyl-2,2,6,6-tetramethyl-4-maleinimido-piperidin

1-Benzyl-2,2,6,6-tetramethyl-4-maleinimido-piperidin

1,3,8-Triaza-2,4-dioxo-3-acryloyl-oxyäthyl-7,7,8,9,9-pentamethyl-spiro-[4,5]-decan

1,3,8-Triaza-2,4-dioxo-3-methacryloyl-oxyäthyl-7,7,8,9,9-pentamethyl-spiro-[4,5]-decan

1,3,8-Triaza-2,4-dioxo-3-n-dodecyl-7,7,9,9-tetramethyl-8-methacryloyl-oxyäthyl-spiro-[4,5]-decan

1,3,8-Triaza-2,4-dioxo-3-methacryloyl-oxyäthyl-7,7,9,9-tetramethyl-8-benzyl-spiro-[4,5]-decan

1,3,8-Triaza-2,4-dioxo-3-n-butyl-7,7,9,9-tetramethyl-8-acryloyl-oxy-äthyl-spiro-[4,5]-decan

1-Benzyl-2,2,6,6-tetramethyl-4-(N-n-butyl)-methacrylamido-piperidin

1,2,2,6,6-Pentamethyl-4-(N-benzyl)-acrylamido-piperidin

1,2,2,6,6-Pentamethyl-4-(N-n-propyl)-acrylamido-piperidin

1,2,2,6,6-Pentamethyl-4-(N-n-propyl)-methacrylamido-piperidin

1-Allyl-2,2,6,6-tetramethyl-4-acryloyloxy-piperidin

1-Allyl-2,2,6,6-tetramethyl-4-methacryloyloxy-piperidin

1,2,2,6,6-Pentamethyl-4-acrylamido-piperidin

1-Benzyl-2,2,6,6-tetramethyl-4-(N-n-butyl)-acrylamido-piperidin

1-Benzyl-2,2,6,6-tetramethyl-4-acrylamido-piperidin

1-[3'-Acryloyloxy-(2'-hydroxy)-propyl]-2,2,6,6-tetramethyl-piperidin

2,2,6,6-Tetramethyl-4-acryloyloxy-piperidin

1-Benzyl-2,2,6,6-tetramethyl-4-acryloyloxy-piperidin.

Monomere der Formel (XVb), welche sich für die Herstellung der erfindungsgemässen copolymeren Additive eignen, sind z.B.:

2-Hydroxy-4-(2'-acryloyloxyäthoxy)-benzophenon

2-Hydroxy-4-(2'-acryloyloxyäthoxy)-benzophenon

2-Hydroxy-4-(2'-methacryloyloxyäthoxy)-benzophenon

2-Hydroxy-4-(2'-acryloyloxy-2'-methyläthoxy)-benzophenon

2-Hydroxy-4-(2'-methacryloyloxyäthoxy)-4'-methyl-benzophenon

2-Hydroxy-4-(2'-acryloylamido-äthoxy)-benzophenon

2-Hydroxy-4-(methacryloyloxy)-benzophenon

BAD ORIGINAL

2-(2'-Hydroxy-3'-methacryloylamidomethyl-5'-cyclohexylphenyl)-benztriazol
2-(2'-Hydroxy-3'-acryloylamido-phenyl)-5-chlor-benztriazol
2-(2'-Hydroxy-3'-acryloylamidomethyl-5'-tert.butylphenyl)-benztriazol
2-(2'-Hydroxy-3'-methacryloylamidomethyl-5'-methyl-phenyl)-benztriazol
2-(2'-Hydroxy-5'-acryloylbenzylamidophenyl)-benztriazol
2-(2'-Hydroxy-4'-[2"-methacryloyloxy-äthoxy]phenyl)-benztriazol
2-(2,4-Di-hydroxy-3'-acryloylamidomethyl-phenyl)-benztriazol
4-Acryloyloxy-benzyliden-malonsäure-diäthylester
4-Methacryloyl-äthoxy-benzyliden-malonsäure-di-n-octylester
[4-(Acryloyloxy-äthoxy)-α-methylbenzyliden]-cyanessigsäuremethylester.
2-Cyano-3-methyl-3-(4-acryloyloxy-phenyl)-acrylsäure-äthylester
4-Hydroxy-benzylidenmalonsäure-diäthylester
(4-Acryloyloxy-benzyliden)-cyanessigsäure-(1,2,2,6,6-pentamethyl-piperidin-4-yl)ester

Für die Darstellung der copolymeren Additive mit der wiederkehrenden Struktureinheit der Formel (I) eignen sich vor allem die unter den Namen radikalische und ionische Homo- bzw. Copolymerisation bekannten Reaktionen. Die Polymerisation wird in bekannter Weise durch Initiatoren und Regler oder Kettenabbrecher gesteuert. Dadurch ist es möglich, zu Polymeren gewünschten Molekulargewichts zu gelangen. Die Reaktion kann in Substanz, in Lösung, in Dispersion, in Emulsion, in Suspension, oder als sogenannte Perlpolymerisation durchgeführt werden.

Für die radikalische Homo- bzw. Copolymerisation eignen sich vor allem Perverbindungen, Azoverbindungen sowie Redoxsysteme als Initiatoren. Gebräuchliche organische oder anorganische Perverbindungen sind u.a. Hydroperoxide, Dialkylperoxide, Diacylperoxide, Perester oder Peroxodisulfate. Beispiele für Perverbindungen sind Wasserstoffperoxid, Kaliumperoxodisulfat, Cumolhydroperoxid, Di-t-butylperoxid, Aethylmethylketonperoxid, Cyclohexanonperoxid, Perbenzoesäure-t.butylester oder gegebenenfalls durch Chlor oder Brom substituiertes

Dibenzoylperoxid. Als Azoverbindungen eignen sich insbesondere solche, bei denen die Azogruppe beiderseits an tertiäre Kohlenstoffatome gebunden ist, die neben Alkylgruppen noch Nitril- oder Estergruppen tragen. $\alpha,\alpha'$-Azoisobuttersäuredinitril und Perbenzoesäure-tert.-butylester sind wichtige Vertreter dieser Initiatorenklasse. Wird die Polyreaktion mittels eines Redoxsystems ausgelöst, so eignen sich als Oxidationsmittel organische oder anorganische Perverbindungen und als Reduktionsmittel entweder Metallionen niedriger Wertigkeitsstufe oder metallfreie Verbindungen, die sich leicht oxydieren lassen. Beispiele für Oxidationsmittel sind Wasserstoffperoxid, Peroxidisulfate oder Diacylperoxide. Als Reduktionsmittel kommen $Ag^{+}$, $Fe^{2+}$, $Ti^{3+}$, Hydrogensulfit, Sulfit, Thiosulfat, Mercaptane, Sulfine, Amine, Endiole (Zucker), Benzoin/$Fe^{2+}$ oder Hydrogensulfit/$Fe^{2+}$ in Frage. Während bei den Perverbindungen und den Azoverbindungen die Art des Initiators lediglich die Polymerisationsgeschwindigkeit, den mittleren Polymerisationsgrad, die Art der Endgruppen oder die Zahl der Verzweigungen, nicht aber die Polymerisierbarkeit beeinflusst, ist nicht jedes Redoxsystem für jede ungesättigte Verbindung geeignet.

Das Molekulargewicht des Polymers steuert man am einfachsten mittels geeigneter Regler. Beispiele sind Mercaptane, wie n-Butylmercaptan oder Dodecylmercaptan und andere organische Schwefelverbindungen, wie Diisopropylxanthogendisulfid, sowie aliphatische Aldehyde und Acetale oder Allylverbindungen, wie Allylalkohol. Die Reaktions-Temperaturen sind dem Fachmann bekannt und betragen für die radikalische Polymerisation, welche bevorzugt angewendet wird, je nach Art der verwendeten Komponenten 40° C bis 160° C, bevorzugt 60° C bis 130° C.

Erfolgt die Polymerisation ionisch, so kann es sich um kationische, bevorzugt jedoch um anionische Polymerisation handeln.

Als Initiatoren kommen metallorganische Verbindungen, wie Diäthylzink oder Diisobutylzink, Naphthalinnatrium, n-Amylnatrium, Cyclopentadienylnatrium, n-Butyllithium oder Triäthylaluminium in Frage. Als Initiatoren wirken ferner Basen, wie Alkalimetallhydroxide, -alkoholate und -amide. Anstelle der bei der radikalischen Polymerisation

BAD ORIGINAL

angewandten Reglern, werden bei der ionischen Polymerisation Stoffe eingesetzt, die mit dem wachsenden Kettenende reagieren; hierzu zählen z.B. Wasser, Alkohole, Säuren und Amine. Die Temperatur bei dieser Reaktionsvariante beträgt von -100° C bis +200° C, bevorzugt -20° C bis +150° C und ist dem Fachmann für den jeweils gewünschten Polymertyp bekannt.

Es ist ferner ebenfalls möglich, als Initiatoren für die Polymerisation Wärme, Licht oder andere energiereiche Strahlung zu verwenden. Die Reaktion läuft dabei nach dem radikalischen Mechanismus ab. Bei der photoinitiierten Polymerisation sind als Katalysatoren z.B. Benzoinäther, Benzilketale, ω-Dialkoxy-acetophenon-Derivate oder aromatische Keton-Amin-Kombinationen geeignet.

Je nach Wahl der Copolymerisationsparameter erhält man auf diese Weise statistische Copolymere oder auch Blockcopolymere.

Währenddem die Copolymerisation zweier Monomerer und die damit verbundenen Effekte besonders gut untersucht worden sind, ist es in gewissen Fällen auch von Nutzen, Polymere aus drei oder mehr polymerisierbaren Verbindungen einzusetzen.

Die Verbindungen mit der wiederkehrenden Struktureinheit der Formel (I) können gemäss der vorliegenden Erfindung als Stabilisatoren für Kunststoffe gegen deren Schädigung durch Einwirkung von Sauerstoff, Wärme und Licht verwendet werden. Beispiele für solche Kunststoffe sind die in der DE-OS 2 456 864 auf den Seiten 12 bis 14 aufgeführten Polymeren.

Von besonderer Bedeutung ist die Stabilisierung von Polyolefinen, Styrolpolymerisaten, Polyamiden und von Polyurethanen, für die sich die Verbindungen der Formel (I) hervorragend eignen. Beispiele hierfür sind Polyäthylen hoher und niedriger Dichte, Polypropylen, Aethylen-Propylen-Copolymerisate,Polyvinylchlorid,Polystyrol,Styrol-Butadien-Acrylnitril-Tercopolymerisate,Mischungen von Polyolefinen oder von Styrolpolymerisaten,Polyurethane auf Polyäther- oder Polyesterbasis in Form von Lacken,Fäden, Folien,Platten, Filmen, Elastomeren oder Schaumstoffen,

Lacken auf Basis thermoplastischer Acrylharze, vernetzbarer Acryl-, Polyester- bzw. Alkydharze sowie zweikomponenten Polyurethanharze.

Die homopolymeren oder copolymeren Stabilisatoren werden den Kunststoffen in einer Konzentration von 0,05 bis 5 Gew.-%, berechnet auf das zu stabilisierende Material, zugesetzt. Vorzugsweise werden 0,1 bis 2,5 Gew.-% der Verbindungen, berechnet auf das zu stabilisierende Material, in dieses eingearbeitet.

Die Einarbeitung kann nach der Polymerisation erfolgen, beispielsweise durch Einmischen der Verbindungen und gegebenenfalls weiterer Additive in die Schmelze nach den in der Technik üblichen Methoden, vor oder während der Formgebung, oder auch durch Aufbringen der gelösten oder dispergierten Verbindungen auf das Polymere, gegebenenfalls unter nachträglichem Verdunsten des Lösungsmittels.

Die neuen Verbindungen können auch in Form eines Masterbatches, der diese Verbindungen beispielsweise in einer Konzentration von 2,5 bis 25 Gew.-% enthält, den zu stabilisierenden Kunststoffen zugesetzt werden.

Die Erfindung betrifft daher auch die durch Zusatz von 0,01 bis 5 Gew.-% einer Verbindung der Formel (I) stabilisierten Kunststoffe, die gegebenenfalls noch andere bekannte und übliche Zusätze enthalten können. Die so stabilisierten Kunststoffe können in verschiedenster Form angewendet werden, z.B. als Folien, Fasern, Bändchen, Profile oder als Bindemittel für Lacke, Klebemittel oder Kitte.

Als weitere Additive, mit denen zusammen die erfindungsgemäss verwendbaren Stabilisatoren eingesetzt werden können, sind beispielsweise zu nennen:

Antioxydantien, wie 2,6-Dialkylphenole , Derivate von alkylierten Hydrochinonen, hydroxylierte Thiodiphenyläther, Alkyliden-bisphenole, O-, N- und S-Benzylverbindungen, hydroxybenzylierte Malonester, Hydroxybenzyl-Aromaten, 2-Triazinverbindungen, Amide der β-(3,5-Di-tert.-butyl-4-hydroxyphenyl)-propionsäure, Ester der β-(3,5-Di-tert.-butyl-4-hydroxyphenyl)-propionsäure, Ester der β-(5-tert.-Butyl-4-hydroxy-3-methylphenyl)-propionsäure, Ester der 3,5-Di-tert.-butyl-4-hydroxyphenylessigsäure, Acylaminophenole, Benzylphosphonate, Aminoarylderivate, UV-Absorber und Lichtschutzmittel, wie 2-(2'-Hydroxyphenyl)-benztriazole, 2,4-Bis-(2'-hydroxyphenyl)-6-alkyl-s-triazine, 2-Hydroxybenzophenone, 1,3-Bis-(2'-hydroxybenzoyl)-benzole, Ester von gegebenenfalls substituierten Benzoesäuren, Acrylate, des weiteren Nickelverbindungen, sterisch gehinderte Amine, Oxalsäurediamide, Metalldesaktivatoren, Phosphite, peroxidzerstörende Verbindungen, Polyamidstabilisatoren, basische Co-Stabilisatoren, PVC-Stabilisatoren, Nukleierungsmittel oder sonstige Zusätze, wie z.B. Weichmacher, Gleitmittel, Emulgatoren, Füllstoffe, Russ, Asbest, Kaolin, Talk, Glasfasern, Pigmente, optische Aufheller, Flammschutzmittel, Antistatica.

Beispiele für weitere Additive, mit denen zusammen die erfindungsgemäss verwendbaren Stabilisatoren eingesetzt werden können, finden sich in der DE-OS 2 427 853 auf Seiten 18 bis 24.

Die folgenden Beispiele dienen zur Illustration der vorliegenden Erfindung:

## Beispiel 1

In einem mit Rückflusskühler, Tropftrichter, Thermometer, Gaseinleitungsrohr und Rührer versehenen Reaktionsgefäss wurden (nach Spülen mit Argon) 16,3 g (0,04 Mol) 2-[(2'-Hydroxy-3'-acrylamidomethyl-5'--tert.octyl)-phenyl]-benzotriazol (Schmelzpunkt 162 bis 163° C) und 13,5 g destilliertes 4-Acryloyloxy-1,2,2,6,6-pentamethyl-piperidin (0,06 Mol) (Kp.: 52 bis 54°/0,02 Torr) in 120 ml reinem Benzol vorgelegt und die Lösung rasch auf 77 bis 78° C erwärmt. Bei dieser Temperatur tropfte man unter Rühren innerhalb von ca. 5 Minuten die Lösung von 0,15 g α,α'-Azoisobutyronitril (gelöst in 5 ml reinem Benzol) zu, wobei die Innentemperatur auf 79 bis 80° C anstieg. Die Polymerisationslösung wurde anschliessend weitere 16 Stunden bei 77 bis 78° C gerührt. Hierauf versetzte man nochmals mit 0,15 g α,α-Azoisobutyronitril in 5 ml Benzol und rührte weitere 24 Stunden bei ca. 77 bis 78° C.

Zur Isolierung des Copolymerisates wurde die Reaktionslösung langsam und unter kräftigem Turbinieren in 600 ml Methanol von -30° C eingegossen und durch Kühlung bei dieser Temperatur gehalten. Nach ca. 30 Minuten Turbinieren wurde das feinpulvrig ausgefallene Copolymerisat rasch abfiltriert, mit Methanol von -30° C nachgewaschen, abgesaugt und im Vakuumschrank erst bei Raumtemperatur, anschliessend bei 80 bis 90° C getrocknet. Das so erhaltene Copolymerisat weist einen Erweichungspunkt von ca. 145° C und ein mittleres Molekulargewicht ($\bar{M}_n$) von 31'600 auf. $\lambda_{max.}$ 304 und 340 nm.
(Additiv 1)

## Beispiele 2 bis 5

Analog der im Beispiel 1 beschriebenen Methode wurden folgende Copolymerisate hergestellt.
(Dabei genügten of bedeutend geringere Reaktionszeiten als im Beispiel 1 angegeben):

| Beispiel | Monomer | Molverhältnis A:B | Lösungsmittel | Copolymer: Ep=Erweichungspunkt $\overline{M}_n$=Molgewicht |
|---|---|---|---|---|
| 2 | (A) 1,2,2,6,6-Pentamethyl-4-acryloyloxypiperidin (B) 2-(2'-Hydroxy-3'-acryloylamidomethyl-5'-sec.butylphenyl)-benztriazol | 3:2 | Benzol | Ep: ~ 145° C<br>$\overline{M}_n$: 52'000 |
| 3 | (A) 1,2,2,6,6-Pentamethyl-4-acryloyloxypiperidin (B) 2-(2'-Hydroxy-3'-methacryloylamidomethyl-5'-methylphenyl)-benztriazol | 2:1 | Benzol | Ep: ~ 120° C<br>$\overline{M}_n$: ~ 7'100 |
| 4 | (A) 1,2,2,6,6-Pentamethyl-4-acryloyloxypiperidin (B) 2-(2'-Hydroxy-3'-acryloylamidomethyl-5'-methylphenyl)-benztriazol | 2:1 | Dioxan | Ep: ~ 150° C<br>$\overline{M}_n$: ~ 10'000 |
| 5 | (A) 1-Benzyl-2,2,6,6-tetramethyl-4-acryloyloxy-piperidin (B) 2-Hydroxy-4-(2'-acryloyloxy-äthoxy)-benzophenon | 1:1 | Benzol | Ep: ~ 108° C (Schmelzpunkt: ~ 130 bis 132° C)<br>$\overline{M}_n$: 11'400 |

| Beispiel | Monomer | Molverhältnis A:B | Lösungsmittel | Copolymer Ep:Erweichungspunkt $M_n$=Molgewicht |
|---|---|---|---|---|
| 6 | (A) 1-Acryloyloxyäthyl-2,2,6,6-tetramethyl-piperidin<br>(B) 2-(2'-Hydroxy-3'-acryloylamidomethyl-5'-methylphenyl)-benztriazol | 3:2 | Dioxan | Ep: ~ 125°C<br>$\overline{M}_n$: 3'300 |
| 7 | (A) 1,2,2,6,6-Pentamethyl-4-acryloyloxy-piperidin<br>(B) 2-(2'-Hydroxy-3'-acryloylamidomethyl-5'-t.butylphenyl)-benztriazol | 1:1 | Benzol/Dioxan | Ep: ~ 175°C<br>$\overline{M}_n$: 13'500 |
| 8 | (A) 1-Acryloyloxyäthyl-2,2,6,6-tetramethyl-piperidin<br>(B) 2-(2'-Hydroxy-3'-acryloylamidomethyl-5'-t-butylphenyl)-benztriazol | 1:1 | Benzol/Dioxan | Ep: ~ 150°C<br>$\overline{M}_n$ 7'600 |
| 9 | (A) 1-Acryloyloxyäthyl-2,2,6,6-tetramethyl-piperidin<br>(B) 2-(2'-Hydroxy-3'-acryloylamidomethyl-5'-methylphenyl)-benztriazol<br>(C) Acrylsäureäthylester | 1:1:1 | Dioxan | Ep: ~ 100°C<br>$\overline{M}_n$: 6'400 |
| 10 | (A) 1,2,2,6,6-Pentamethyl-4-acryloyloxy-piperidin<br>(B) 2-Hydroxy-4-acryloyl-oxyäthoxy-benzophenon | 6:5 | Benzol | Ep: ~ 108 °C<br>$\overline{M}_n$: 11'400 |

| Beispiel | Monomer | Molverhältnis A:B | Lösungsmittel | Copolymer Ep:Erweichungspunkt $\overline{M}_n$=Molgewicht |
|---|---|---|---|---|
| 11 | (A) 1-Acryloyloxyäthyl-2,2,6,6-tetramethyl-piperidin (B) 2-Hydroxy-4-acryloyloxy-benzophenon | 4:5 | Benzol | Ep: ~ 135°C $\overline{M}_n$: 6'300 |
| 12 | (A) 1-Acryloyloxyäthyl-2,2,6,6-tetramethyl-piperidin (B) 2-Hydroxy-4-acryloyloxyäthoxy-benzophenon | 1:1 | Benzol | Ep: ~ 86°C $\overline{M}_n$: 6'100 |
| 13 | (A) 1-n-Butyl-2,2,6,6-tetramethyl-4-acryloyloxy-piperidin (B) 2-Hydroxy-4-acryloyloxyäthoxy-benzophenon | 1:1 | Benzol | Ep: ~ 110°C $\overline{M}_n$: 12'500 |
| 14 | (A) 1,2,2,6,6-Pentamethyl-4-acryloyloxy-piperidin (B) 2-Hydroxy-4-acryloyloxyäthoxy-benzophenon | 1:1 | Benzol | Ep: ~ 105°C $\overline{M}_n$: 34'600 |
| 15 | (A) 1,2,2,6,6-Pentamethyl-4-acryloyloxy-piperidin (B) 2-Hydroxy-4-acryloyloxyäthoxy-benzophenon | 1:1 | Benzol | Ep: ~ 82°C $\overline{M}_n$: 4'400 |
| 16 | (A) 1-Acryloyloxyäthyl-2,2,6,6-tetramethyl-piperidin (B) 2-Hydroxy-4-acryloyloxy-2'-hydroxy-propoxy-benzophenon | 1:1 | Benzol | Ep: ~ 74°C $\overline{M}_n$: 3'700 |

| Beispiel | Monomer | Molverhältnis A:B | Lösungsmittel | Copolymer Ep:Erweichungspunkt $\overline{M}_n$=Molgewicht |
|---|---|---|---|---|
| 17 | (A) 1,2,2,6,6-Pentamethyl-4-acryloyloxypiperidin (B) 2-Hydroxy-4-acryloyloxybenzophenon | 3:2 | Benzol | Ep: ~ 140°C $\overline{M}_n$: 16'000 |
| 18 | (A) 1,3,8-Triaza-2,4-dioxo-3-n-butyl-7,7,9,9-tetramethyl-8-acryloyloxyäthyl-spiro-[4,5]-decan (B) 2-Hydroxy-4-acryloyloxybenzophenon | 1:1 | Benzol | Ep: ~ 132°C $\overline{M}_n$ 15'200 |
| 19 | (A) 1-Acryloyloxyäthyl-2,2,6,6-tetramethylpiperidin (B) 4-Acryloyloxy-benzylidencyanessigsäureäthylester | 1:1 | Benzol | Ep:~ 95°C $\overline{M}_n$: 5'800 |
| 20 | (A) 1-Benzyl-2,2,6,6-tetramethyl-4-acryloyloxy-piperidin (B) 4-Acryloyloxybenzylidencyanessigsäure-äthylester | 1:1 | Benzol | Ep: ~ 125°C $\overline{M}_n$: 4'200 |
| 21 | (A) 1-Acryloyloxy-2,2,6,6-tetramethylpiperidin (B) 4-Acryloyloxybenzylidenmalonsäuredimethylester | 1:1 | Benzol | Ep: ~ 90°C $\overline{M}_n$: 23'800 |
| 22 | (A) 1,2,2,6,6-Pentamethyl-4-methacryloyloxy-piperidin (B) 4-Acryloyloxy-benzilidenmalonsäure-dimethylester | 1:1 | Benzol | Ep: ~ 120°C $\overline{M}_n$: 10'700 |

| Bei-spiel | Monomer | Molver-hältnis A:B | Lösungs-mittel | Copolymer Ep:Erweichungspunkt $\overline{M}_n$=Molgewicht |
|---|---|---|---|---|
| 23 | (A) 1,2,2,6,6-Pentamethyl-4-acryloyloxy-piperidin (B) 4-Acryloyloxy-benzylidene-cyanessigsäure-(1',2',2',6',6'-pentamethylpiperidin-4'-yl)ester | 1:1 | Benzol | Ep: ~ 150°C $\overline{M}_n$: 5'800 |
| 24 | (A) 1-Acryloyloxyäthyl-2,2,6,6-tetramethyl-piperidin (B) 2-Cyano-3-methyl-3-(4'-acryloyloxy-phenyl)-acrylsäure-äthylester | 1:1 | Benzol | Ep: ~ 105°C $\overline{M}_n$: 6'900 |
| 25 | (A) 1-Acryloyloxyäthyl-2,2,6,6-tetramethyl-piperidin (B)4-Acryloyloxyäthoxy-benzyliden-cyanessigsäure-dimethylester | 2:1 | Benzol | Ep: ~ 76°C $\overline{M}_n$: 11'500 |
| 26 | (A) 1-Acryloyloxyäthyl-2,2,6,6-tetramethyl-piperidin (B) 4-Acryloyloxy-benzyliden-malonsäure-di-(1',2',2',6',6'-pentamethylpiperidin-4'-yl)-ester | 3:2 | Benzol | Ep : ~ 102°C $\overline{M}_n$ : 7'000 |

## Patentansprüche

1. Seitenständige N-heterocyclische Ringe tragende copolymere Verbindungen mit der wiederkehrenden Struktureinheit der Formel (I)

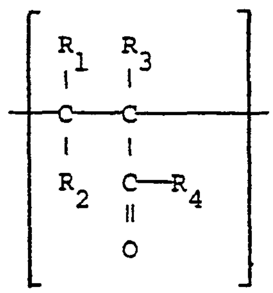

(I) ,

worin $R_1$ und $R_2$ Wasserstoff sind, und $R_3$ Wasserstoff oder $C_1$-$C_4$ Alkyl bedeutet, und $R_4$ ein N-heterocyclischer Substituent der Formeln (II), (III), (IV) oder (V)

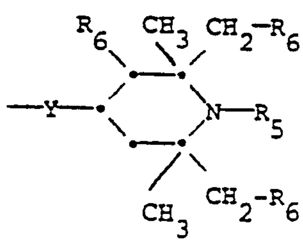

,

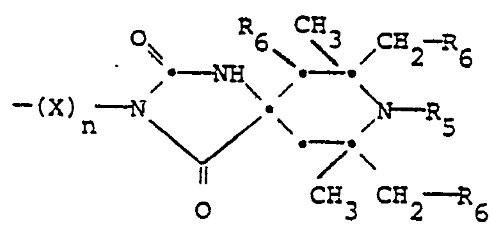

(II)

(III)

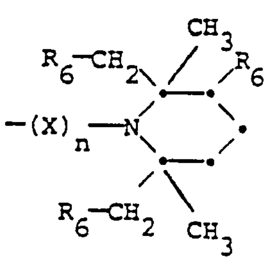

,

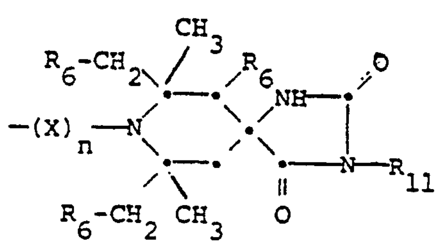

(IV)

(V)

bedeutet, worin $R_5$ Wasserstoff, Oxyl, $C_1$-$C_{18}$ Alkyl, $C_3$-$C_8$ Alkenyl, $C_3$-$C_6$ Alkinyl, $C_7$-$C_{12}$ Aralkyl, $C_2$-$C_{21}$ Alkoxyalkyl, Cyanomethylen, eine aliphatische Acylgruppe mit 1 bis 4 Kohlenstoffatomen oder eine Gruppe $-CH_2COOR_7$ ist, wobei $R_7$ $C_1$-$C_{12}$ Alkyl, $C_3$-$C_8$ Alkenyl, Phenyl, $C_7$-$C_8$ Aralkyl oder Cyclohexyl bedeutet, und $R_6$ Wasserstoff oder Methyl bedeutet, und Y -O- oder $-\overset{|}{N}-R_9$ ist, wobei $R_9$ Wasserstoff, $C_1$-$C_{12}$ Alkyl, $C_5$-$C_8$ Cycloalkyl oder $C_7$-$C_{12}$ Aralkyl ist, und X eine Gruppe der Formel $-O-CH(R_{10})-CH_2-$ (VI) ist, worin $R_{10}$ Wasserstoff, Methyl, Aethyl, Phenoxymethyl oder Phenyl bedeutet, und n O oder 1 ist, und $R_{11}$ $C_1$-$C_{18}$ Alkyl, Cyclohexyl, Phenyl, Benzyl oder eine Gruppe der Formel $-CH_2-COOR_{12}$ (VII) bedeutet, wobei $R_{12}$ $C_1$-$C_8$ Alkyl ist, und ferner $R_4$ eine der Gruppen der Formel (VIII), (IX), (X) oder (XI)

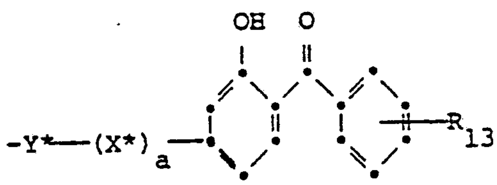

(VIII)

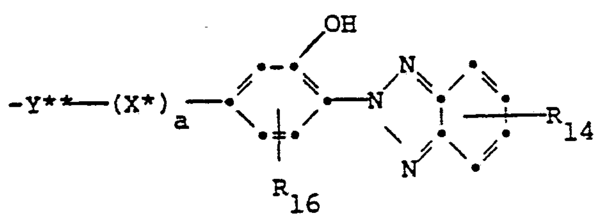

(IX)

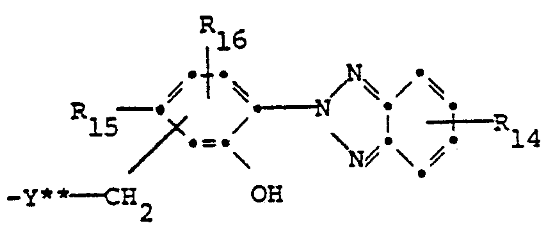

(X)

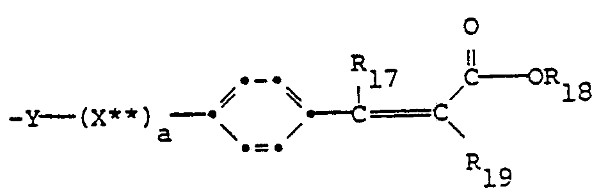

(XI)

bedeutet, worin Y und Y** die oben für Y definierte Bedeutung haben, und Y* -O- oder $-\overset{|}{N}-R_9*$ ist, wobei $R_9*$ Wasserstoff oder $C_1-C_8$ Alkyl ist, und X* eine Gruppe der Formel $-CH(R_{10})-(CH_2)_b-O-$ (XII) bedeutet, b 1 oder 2 ist, und $R_{10}$ die oben angegebene Bedeutung hat, und a 0 oder 1 ist, und $R_{13}$ Wasserstoff, Methyl oder Chlor bedeutet, und $R_{14}$ Wasserstoff, Methyl, Methoxy oder Chlor bedeutet, und $R_{15}$ Wasserstoff oder Hydroxy bedeutet, und $R_{16}$ H, $C_1-C_{12}$ Alkyl, $C_5-C_8$ Cycloalkyl, Phenyl, $C_7-C_{12}$ Aralkyl oder Chlor ist, und X** eine Gruppe der Formel (XII) ist, worin $R_{10}$ die oben angegebene Bedeutung hat, und b 1 ist, und $R_{17}$ Wasserstoff oder $C_1-C_4$ Alkyl bedeutet, und $R_{18}$ $C_1-C_{12}$ Alkyl, $C_5-C_8$ Cycloalkyl oder eine Gruppe der Formel (XIII)

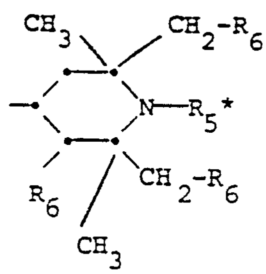

(XIII) ,

worin $R_5*$ Wasserstoff, $C_1-C_8$ Alkyl, $C_3-C_8$ Alkenyl, $C_7-C_{12}$ Aralkyl oder eine aliphatische Acylgruppe mit 1 bis 4 Kohlenstoffatomen ist, und $R_6$ die oben angegebene Bedeutung hat, und $R_{19}$ -CN oder eine Gruppe der Formel $-COOR_{18}$ (XIV) ist, worin $R_{18}$ die oben angegebene Bedeutung hat, mit der Bedingung, dass im copolymeren Molekül mit der wiederkehrenden Struktureinheit I der Substituent $R_4$ mindestens einmal (A) eine der Gruppen der Formel (II), (III), (IV) oder (V) und mindestens einmal (B) eine der Gruppen der Formeln (VIII), (IX), (X) oder (XI) bedeutet, wobei (A):(B) im Verhältnis von 50:1 bis 1:50 stehen.

2. Copolymere Verbindungen gemäss Anspruch 1 mit der wiederkehrenden Struktureinheit der Formel (I), worin $R_1$ und $R_2$ Wasserstoff sind, und $R_3$ Wasserstoff oder Methyl ist, und $R_4$ eine Gruppe der Formeln (II), (III), (IV) oder (V) ist, worin $R_5$ Wasserstoff, $C_1-C_{12}$ Alkyl, Allyl, Propargyl, Benzyl, Acetyl oder $C_2-C_6$ Alkoxyalkyl bedeutet, und $R_6$ Wasserstoff oder Methyl ist, und Y -O- oder

$-\overset{|}{N}-R_9$ ist, worin $R_9$ Wasserstoff, $C_1$-$C_{12}$ Alkyl, Cyclohexyl oder Benzyl ist und X eine Gruppe der Formel (VI) bedeutet, worin $R_{10}$ Wasserstoff, Methyl oder Aethyl ist, und n O oder 1 ist, und $R_{11}$ $C_1$-$C_{12}$ Alkyl, Allyl oder Benzyl bedeutet, und ferner $R_4$ eine Gruppe der Formeln (VIII), (IX), (X) oder (XI) bedeuten, worin Y und Y** die oben für Y angegebene Bedeutung haben, und Y* -O- oder $-\overset{|}{N}-R_9$* ist, worin $R_9$* Wasserstoff oder $C_1$-$C_4$ Alkyl ist, und X* eine Gruppe der Formel (XII) ist, worin $R_{10}$ die oben definierte Bedeutung hat, und b 1 oder 2 ist, und a O oder 1 ist, $R_{13}$ Wasserstoff, Methyl oder Chlor ist, und $R_{14}$ und $R_{15}$ Wasserstoff sind, und $R_{16}$ H, $C_1$-$C_8$ Alkyl, Cyclohexyl, Phenyl, $C_7$-$C_9$ Aralkyl oder Chlor ist, und X** und $R_{17}$ die oben definierte Bedeutung haben und $R_{18}$ $C_1$-$C_8$ Alkyl, Cyclohexyl oder eine Gruppe der Formel (XIII) ist, worin $R_5$* Wasserstoff, $C_1$-$C_8$ Alkyl, Allyl, Benzyl oder Acetyl ist, und $R_6$ die angegebene Bedeutung hat, und $R_{19}$ -CN oder eine Gruppe der Formel (XIV) ist, worin $R_{18}$ die oben angegebene Bedeutung hat.

3. Copolymere Verbindungen gemäss Anspruch 1 mit der wiederkehrenden Struktureinheit der Formel (I), worin $R_1$ und $R_2$ Wasserstoff sind, und $R_3$ Wasserstoff oder Methyl bedeutet, und $R_4$ eine Gruppe der Formeln (II) oder (IV) ist, worin $R_5$ Wasserstoff, $C_1$-$C_8$ Alkyl, Allyl, Benzyl oder Acetyl ist, und $R_6$ Wasserstoff bedeutet, und Y -O- oder $-\overset{|}{N}-R_9$ ist, worin $R_9$ Wasserstoff oder $C_1$-$C_4$ Alkyl bedeutet, und X eine Gruppe der Formel (VI) ist, worin $R_{10}$ Wasserstoff oder Methyl und n O oder 1 bedeutet, und ferner $R_4$ eine Gruppe der Formeln (VIII), (IX), (X) oder (XI) ist, worin Y die oben angegebene Definition besitzt, und Y** $-\overset{|}{N}-R_9$ ist, worin $R_9$ die oben angegebene Bedeutung hat, und Y* -O- ist, und X* und X** eine Gruppe der Formel (XII) ist, worin $R_{10}$ die oben angegebene Bedeutung hat und b 1 ist, und a O oder 1 ist, und $R_{13}$, $R_{14}$ und $R_{15}$ Wasserstoff sind, und $R_{16}$ H, $C_1$-$C_8$ Alkyl oder $C_7$-$C_9$ Aralkyl bedeutet, und $R_{17}$, falls $R_{19}$ -CN ist, $C_1$-$C_4$ Alkyl oder, falls $R_{19}$ eine Gruppe der Formel (XIV) ist, Wasserstoff bedeutet, und $R_{18}$ $C_1$-$C_8$ Alkyl oder eine Gruppe der Formel (XIII) bedeutet, worin $R_5^*$ Wasserstoff, $C_1$-$C_4$ Alkyl, Benzyl oder Acetyl ist, und

$R_4$ die oben erwähnte Definition besitzt, und $R_{19}$ -CN oder eine Gruppe der Formel (XIV) ist, worin $R_{18}$ die oben erwähnte Bedeutung hat.

4. Copolymere Verbindungen gemäss Anspruch 1 mit der wiederkehrenden Struktureinheit der Formel (I), worin $R_1$ und $R_2$ Wasserstoff sind, und $R_3$ Wasserstoff oder Methyl ist, und $R_4$ eine Gruppe der Formeln (II) oder (IV) ist, worin $R_5$ Wasserstoff, $C_1$-$C_4$ Alkyl, Benzyl oder Acetyl ist, und $R_6$ Wasserstoff bedeutet, und Y -O- oder -$NR_9$ ist, worin $R_9$ Wasserstoff oder $C_1$-$C_4$ Alkyl ist und X eine Gruppe der Formel (VI) ist, worin $R_{10}$ Wasserstoff oder Methyl und n 0 oder 1 ist, und $R_4$ eine Gruppe der Formeln (VIII), (X) oder (XI) ist, worin Y und Y* -O- sind, und Y** -N-$R_9$ ist, wobei $R_9$ die oben angegebene Bedeutung hat, und X* und X** eine Gruppe der Formel (XII) sind, worin $R_{10}$ die oben angegebene Bedeutung hat, und b 1 ist, und a 0 oder 1 ist, und $R_{13}$, $R_{14}$ und $R_{15}$ Wasserstoff sind, und $R_{16}$ H, $C_1$-$C_8$ Alkyl oder $C_7$-$C_9$ Aralkyl ist, und $R_{17}$, falls $R_{19}$ -CN ist, Methyl oder, falls $R_{19}$ eine Gruppe der Formel (XIV) ist, Wasserstoff bedeutet, und $R_{18}$ $C_1$-$C_8$ Alkyl oder eine Gruppe der Formel (XIII) ist, worin $R_5$* Wasserstoff oder Methyl bedeutet, und $R_6$ Wasserstoff ist, und $R_{19}$ -CN oder eine Gruppe der Formel (XIV) bedeutet, worin $R_{18}$ die oben erwähnte Bedeutung hat.

5. Copolymere Verbindungen gemäss Anspruch 1 mit der wiederkehrenden Struktureinheit der Formel (I), worin $R_1$ und $R_2$ Wasserstoff sind, und $R_3$ Wasserstoff oder Methyl ist, und $R_4$ eine Gruppe der Formeln (II) oder (IV) ist, worin $R_5$ Wasserstoff, Methyl oder Acetyl bedeutet, und $R_6$ Wasserstoff ist, und Y -O- oder -N-$R_9$ ist, worin $R_9$ Wasserstoff oder $C_1$-$C_4$ Alkyl bedeutet,und X eine Gruppe der Formel (VI) ist, worin $R_{10}$ Wasserstoff und n 0 oder 1 bedeutet, und ferner $R_4$ eine Gruppe der Formeln (VIII) oder (X) ist, worin Y* -O- ist, und Y** die oben angegebene Bedeutung hat, und X* und X** eine Gruppe der Formel (XII) sind, worin $R_{10}$ die oben angegebene Bedeutung hat, und b 1 ist, und a 0 oder 1 ist, und $R_{13}$, $R_{14}$ und $R_{15}$ Wasserstoff sind, und $R_{16}$ H, $C_1$-$C_8$ Alkyl oder $C_7$-$C_9$ Aralkyl bedeutet.

6. Copolymere Verbindung gemäss Anspruch 1 der Formel I, worin $R_1$, $R_2$ und $R_3$ Wasserstoff sind, und $R_4$ eine Gruppe der Formel IV ist, worin $R_6$ Wasserstoff ist, und X eine Gruppe der Formel VI ist, wobei $R_{10}$ Wasserstoff und n 1 ist, und $R_4$ ferner eine Gruppe der Formel X bedeutet worin $R_{14}$ und $R_{15}$ Wasserstoff sind und $R_{16}$ in p-Position zur Hydroxygruppe t.Butyl ist, und Y** -O- ist.

7. Copolymere Verbindung gemäss Anspruch 1 der Formel I, worin $R_1$, $R_2$ und $R_3$ Wasserstoff sind und $R_4$ eine Gruppe der Formel IV bedeutet, worin $R_6$ Wasserstoff ist, und X eine Gruppe der Formel VI bedeutet, wobei $R_{10}$ Wasserstoff und n 1 ist, und $R_4$ ferner eine Gruppe der Formel VIII ist, worin $R_{13}$ Wasserstoff ist, und X* eine Gruppe der Formel XII ist, wobei a 1, b 0 und $R_{10}$ Wasserstoff bedeutet und ferner Y* -O- ist.

8. Copolymere Verbindung gemäss Anspruch 1 der Formel I, worin $R_1$, $R_2$ und $R_3$ Wasserstoff sind und $R_4$ eine Gruppe der Formel IV ist, worin $R_6$ Wasserstoff ist, und X eine Gruppe der Formel VI bedeutet, wobei $R_{10}$ Wasserstoff und n 1 ist, und $R_4$ ferner eine Gruppe der Formel VIII ist, worin $R_{13}$ Wasserstoff und a 0 ist und Y* -O- bedeutet.

9. Copolymere Verbindungen gemäss Anspruch 1 der Formel I, worin $R_1$, $R_2$ und $R_3$ Wasserstoff sind und $R_4$ eine Gruppe der Formel IV ist, worin $R_6$ Wasserstoff ist, und X eine Gruppe der Formel VI bedeutet, wobei $R_{10}$ Wasserstoff und n 1 ist, und $R_4$ ferner eine Gruppe der Formel XI ist, worin $R_{17}$ Wasserstoff ist, und $R_{18}$ eine Gruppe der Formel XIII ist, worin $R_5^*$ Methyl ist und $R_6$ Wasserstoff bedeutet, und $R_{19}$ eine Gruppe der Formel XIV ist, worin $R_{18}$ die oben angegebene Bedeutung hat und a 0 ist und Y -O- bedeutet.

10. Verwendung der Verbindungen gemäss Anspruch 1 zum Stabilisieren von organischem Material.

11. Organisches Material enthaltend eine Verbindung gemäss Anspruch 1.

12. Verfahren zum Herstellen von Verbindungen gemäss Anspruch 1, dadurch gekennzeichnet, dass man ein Monomer der Formel (XVa)

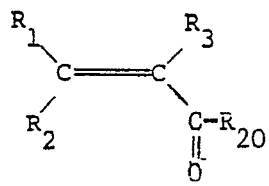

(XVa)

mit einem Monomer der Formel (XVb)

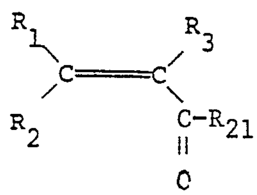

(XVb)

gegebenenfalls in Gegenwart weiterer copolymerisierbarer Komponenten radikalisch, ionisch oder photoinitiert polymerisiert, wobei $R_{20}$ eine Gruppe der Formeln (II), (III), (IV) oder (V) bedeutet, $R_{21}$ eine Gruppe der Formeln (VIII), (IX), (X) oder (XI) ist und diese Gruppen, sowie die Substituenten $R_1$, $R_2$ und $R_3$ die im Anspruch 1 angegebene Bedeutung haben.

Europäisches Patentamt

# EUROPÄISCHER RECHERCHENBERICHT

0010518

Nummer der Anmeldung

EP 79 81 0119

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | DE - A - 2 040 983 (SANKYO)<br>* Patentansprüche 1,2 *<br>-- | 1-9 |
| A | GB - A - 1 112 439 (NAUCHNO-ISSLEDOVATELSKY INSTITUT MONOMEROV DLIA SINTETICHESKOGO KAUCHUKA) | |
| E | EP - A - 0 000 496 (CIBA-GEIGY) | |
| E | EP - A - 0 001 703 (HOECHST)<br>---- | |

KLASSIFIKATION DER ANMELDUNG (Int.Cl. 3)

C 08 F 20/34
C 07 D 211/46
211/58
471/10
C 08 L 101/00

RECHERCHIERTE SACHGEBIETE (Int. Cl. 3)

C 08 F 20/10
20/12
20/34
20/36
20/60
C 07 D 211/46
211/58
471/10

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 08-01-1980 | CAUWENBERG |